# EUROPEAN PATENT SPECIFICATION

(11) **EP 2 803 319 B1**
(45) Date of publication and mention of the grant of the patent: **09.03.2022**
(21) Application number: 13167705.6
(22) Date of filing: 14.05.2013
(51) Int. Cl.: A61B 6/00

(54) **Elevating column and method of controlling elevation thereof**
Hebesäule und Verfahren zur Steuerung des Betriebs davon
Colonne élévatrice et son procédé de commande d'élévation

(43) Date of publication of application: 19.11.2014
(73) Proprietor: Solutions for tomorrow AB, 35595 Tävelsas (SE)
(72) Inventor: Guldstrand, Mattias, 36047 Värends Nöbbele (SE); Yngvesson, Martin Göran Kristoffer, 35595 Tävelsås (SE); Bååt, Jan, 35249 Växjö (SE)
(74) Representative: AWA Sweden AB

(56) References cited:
- WO-A2-2011/130203
- JP-U- S5 283 673
- US-A- 6 052 428
- US-A1- 2006 227 938

## Description

### BACKGROUND OF THE INVENTION

### Field of the Invention

This invention pertains in general to the field of X-ray apparatuses. More particularly the invention relates to mobile X-ray apparatuses and even more particularly to an elevating column of the mobile X-ray apparatus.

### Description of the Prior Art

An elevating column has been disclosed in US2011/0249807 A1, which column enables imaging lower than usual. However, although the range may have been improved, imaging can still not be performed close to the floor. Furthermore, although the apparatus disclosed may be put into a compact shape, it is still heavy. One of the reasons for it being heavy is that a counter-weight is used. Moreover, the elevating column has many components and is thus complicated in its construction. Therefore also control of it may be complicated. In addition, with the counter-weight drive mechanism of the apparatus it may be difficult to obtain sufficient accuracy. Further, it may also be difficult adjusting the elevation fast enough. A mobile x-ray apparatus with an elevating column according to the preamble of claim 1 is disclosed in JP 17125775 U.

Thus, there may be a need for an elevating column with an increased range of reach towards the floor. There may also be a need for a lighter and/or more compact apparatus. Furthermore, there may be a need for a simpler elevating column with fewer components. Moreover, there may be a need for an elevating column, which is simple in construction and/or easy to control. In addition, there may be a need for elevation adjustments with a higher accuracy and/or faster elevation adjustments.

### SUMMARY OF THE INVENTION

Accordingly, embodiments of the present invention preferably seek to mitigate, alleviate or eliminate one or more deficiencies, disadvantages or issues in the art, such as the above-identified, singly or in any combination by providing an elevating column and methods related thereto, according to the appended patent claims.

According to aspects of the invention, a mobile x-ray apparatus comprising an elevating column and methods related thereto are disclosed, whereby elevation adjustments are made with sufficient accuracy and speed.

According to one aspect of the invention, a mobile x-ray apparatus comprising an elevating column for an X-ray apparatus is provided. The X-ray apparatus is mobile. The column is preferably rotationally fixed in relation to a base of the X-ray apparatus. The column has a plurality of segments, including a first segment, which has a first end and an opposite second end. Furthermore, the first segment is arranged at the base with the first end. The column also has a second segment, which has a first end and an opposite second end. Furthermore, the second segment is arranged at its first end with an overlap of the first segment's second end. Moreover the second segment is moveable relative the first segment. The column further includes a first elevation adjustment unit, which unit includes at least a first threaded elongate member fixed at one of the first and second segments. The first elevation adjustment unit also includes a first threaded receiving element arranged at the other of the first and second segments, matingly engaged with the threaded elongate member. The elevating column further includes a connecting segment attaching an arm holding an X-ray tube to said elevating column, said connecting segment is located radially outside said second segment and connected thereto, and said connecting segment is movable radially outside said second segment in a vertical direction.

According to another aspect of the invention, a method of controlling elevation of the elevating column of the mobile x-ray apparatus is provided. The method includes receiving information from position sensors. Furthermore, the method includes sending a first control signal to a first electrical motor. Moreover, an elevation of a moveable second segment of the elevating column is controlled with the first electrical motor by actuating a rotational movement of either a first threaded receiving element or a first threaded elongate member of a first elevation adjustment unit. A second control signal is sent to a second electrical motor. Furthermore, an elevation of a connecting segment, attached to a telescopic arm, of the elevating column is controlled with a second electrical motor by actuating a rotational movement of either a second threaded receiving element or a second threaded elongate member of a second elevation adjustment unit.

According to yet another aspect of the invention, a method of preparing a mobile X-ray apparatus for transport is provided. The method includes controlling elevation of a moveable second segment of an elevating column to a lowest possible position with a first electrical motor. The elevation control is performed by actuating a rotational movement of either a first threaded receiving element or a first threaded elongate member of a first elevation adjustment unit.

The method also includes controlling elevation of a connecting segment, comprising an outer part and an inner part attached to a telescopic arm, of the elevating column to a highest possible position with a second electrical motor. Moreover, the elevation control of the connecting element is performed by actuating a rotational movement of either a second threaded receiving element or a second threaded elongate member of a second elevation adjustment unit. Furthermore, the method includes rotating the outer part horizontally around the inner part until a telescopic arm of the apparatus is positioned on top of a base of the apparatus. In addition, the method may include controlling elevation of the connecting segment by lowering it until the telescopic arm is positioned in a locking position, such as in a hollow space of the base.

Further embodiments of the invention are defined in the dependent claims, wherein features for the second and subsequent aspects of the invention are as for the first aspect mutatis mutandis.

Some embodiments of the invention provide for high precision elevation adjustments.

Some embodiments of the invention also provide for that the operating elevation range of the telescopic arm and the range for image capturing is extended towards very low positions, thus enabling capturing of images at very low positions.

Some embodiments of the invention also provide for that fast and easy rotation with high precision is obtained.

Some embodiments of the invention also provide for faster vertical movement of the column, e.g. if both the second and the connecting segments are operated simultaneously.

Some embodiments of the invention also provide for that the mobile apparatus is easy to transport due to e.g. a compact size and an increased visibility.

Some embodiments of the invention also provide for that the elevation adjustment is split between adjusting the second segment and adjusting the connecting segment.

Some embodiments of the invention also provide for that precision and/or speed of elevation adjustment may be increased.

Some embodiments of the invention also enable movement of the connecting segment in opposite direction as movement of the moveable second segment.

Some embodiments of the invention also provide for an increased stability and avoidance of wobbling of the first threaded elongate member and thus an increase in precision, reliability, safety and life span of the threaded elongate member, the first elevation adjustment unit and the system may be achieved.

Some embodiments of the invention also provide for that the moveable second segment can be height adjusted fast whereas the connecting segment can be used for alignment with high precision. Thus, an increase in adjustment speed and adjustment precision may be achieved at the same time.

Some embodiments of the invention also provide for an extension of the range of reach with the X-ray tube towards the floor.

Some embodiments of the invention also enable the use of larger front wheels, which improves steering, transportation properties and precision.

Some embodiments of the invention also provide for optimization of the range of reach, or optimization of the highest possible point, while maintaining a compact size during transportation.

Some embodiments of the invention also provide for reduction of the number of components, since a sufficient range of reach and a sufficiently high highest possible point can be achieved with only two segments, i.e. the first and second segments, as opposed to three or more.

Some embodiments of the invention also provide for that space inside the column is freed up.

Some embodiments of the invention also provide for that the column may be made smaller, i.e. with a smaller circumference or cross section. Thus the weight may be lowered.

Some embodiments of the invention also provide for that the centre of gravity may be made lower, e.g. if the motor is positioned outside the column and attached to the base instead of inside the column.

Some embodiments of the invention also provide for thinner walls of the column and thus lower weight.

Some embodiments of the invention also provide for an improved stability of the column.

Some embodiments of the invention also enable a compact size during transportation, and thus enable the use of a smaller vehicle, such as a van for transportation.

Some embodiments of the invention also provide for a lower weight of the X-ray apparatus. Thus, it may be easier to transport the apparatus with e.g. a van or another small vehicle instead of transporting it with a truck, and therefore lower fuel consumption and cheaper transportation may be achieved. This may be important if the mobile X-ray apparatus is transported to the patients instead of transporting the patients to an X-ray apparatus.

Some embodiments of the invention also provide for a fast and easy control of elevation of the elevating column and/or the arm.

Some embodiments of the invention also provide for that the speed of adjustment can be increased.

Some embodiments of the invention also provide for a lighter apparatus, since the rotation hub may be made smaller and lighter, since the load may be made smaller due to e.g. the use of a rotationally fixed segment of the column.

Some embodiments of the invention also enable the use of a recessed placement of the column and therefore an extension of the range of reach with the X-ray tube to very low positions and/or space for larger front wheels and/or placement of the motor associated with one of the segments of the column outside the column.

It should be emphasized that the term "includes/including" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which
Fig. 1A is a perspective view of an elevating column;
Fig. 1B is a perspective detail view of limit switches of an elevating column;
Fig. 1C is a perspective detail view of some parts of an elevation adjustment unit for an elevating column;
Fig. 2A is a lateral view of an elevating column;
Fig. 2B is a lateral detail view of some parts of an elevation adjustment unit for an elevating column;
Fig. 2C is a lateral detail view of limit switches of an elevating column;
Fig. 3A is a lateral view of an elevating column with segments, while a moveable second segment is in its least extended position;
Fig. 4A is a lateral view of an elevating column with segments, while a moveable second segment is in its least extended position and a connecting segment is in its lowest position;
Fig. 4B is a lateral detail view of some parts of an elevation adjustment unit for an elevating column with segments, while a moveable second segment is in its least extended position and a connecting segment is in its lowest position;
Fig. 4C is a lateral detail view of limit switches of an elevating column with segments, while a moveable second segment is in its least extended position and a connecting segment is in its lowest position;
Fig. 5A is a lateral view of an elevating column with segments, while a moveable second segment is in its most extended position and a connecting segment is in its highest position;
Fig. 5B is a lateral detail view of some parts of an elevation adjustment unit for an elevating column with segments, while a moveable second segment is in its most extended position and a connecting segment is in its highest position;
Fig. 6 is a lateral view of a mobile X-ray apparatus;
Fig. 7 illustrates steps of a method of controlling elevation of an elevating column; and
Fig. 8 illustrates steps of a method of preparing a mobile X-ray apparatus for transport.

### DESCRIPTION OF THE PREFERRED EMBODIMENTS

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of a mobile X-ray apparatus and in particular to an elevating column of a mobile X-ray apparatus.

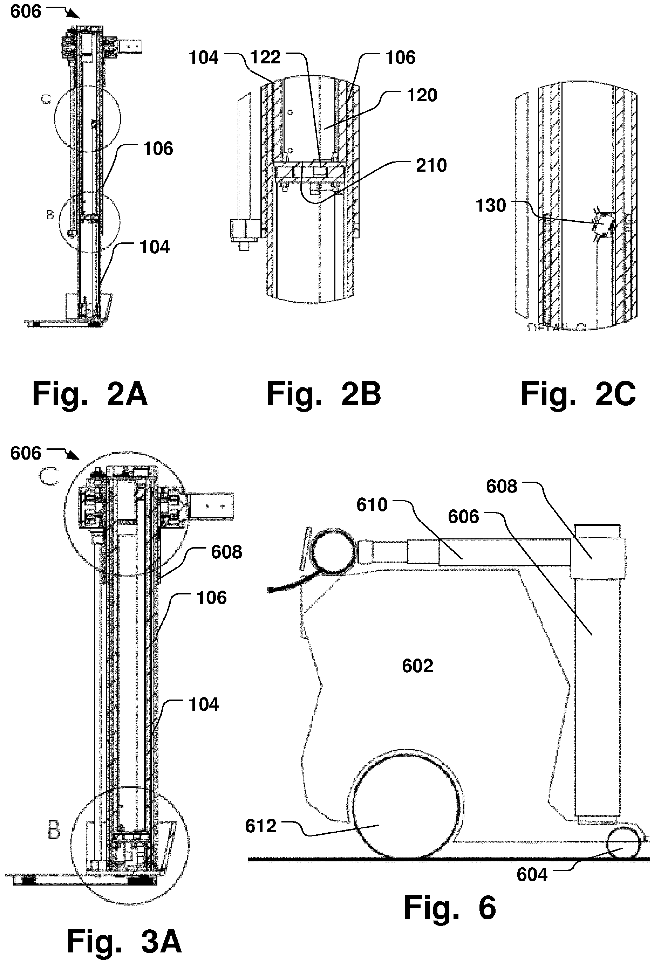

**Fig. 6** shows a mobile X-ray apparatus **600.** The X-ray apparatus **600** has a base **602** and an elevating column **606.** The base **602** has at least one front wheel **604** and at least one rear wheel **612.** Preferably the base **602** has two front wheels and two rear wheels. The front wheels are in this embodiment large, e.g. larger than the normal size of front wheels for mobile X-ray apparatuses and preferably having a diameter which is larger than 12 cm. However, in other embodiments, the front wheels are smaller than the normal size of front wheels for mobile X-ray apparatuses and preferably having a diameter which is smaller than 6 cm. An arm **610** for an X-ray tube, which arm **610** may be telescopic, is attached to the column **606** via a connecting segment **608.**

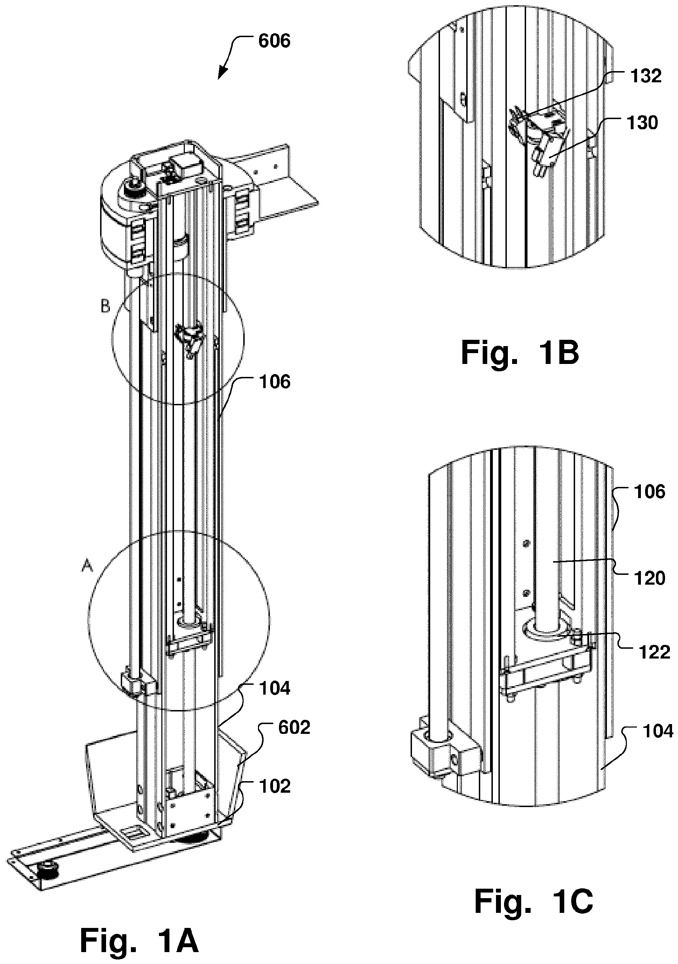

In an embodiment of the invention according to **Fig. 1A****,** which is a perspective view of an elevating column **606,** the elevating column **606** has a second segment **106,** which is moveable, preferably vertically, in relation to a first segment **104.** Thus, the elevating column **606** is in this embodiment rotationally fixed in relation to the base **602** of the X-ray apparatus **600.** However, in other embodiments, the elevating column may move in a rotational direction in relation to the base **602.**

The column **606** has a plurality of segments. It has a second segment **106** having a first end and an opposite second end. The second segment **106** is positioned with the first end at the top and with the opposite second end at the bottom. Furthermore, the column **606** has a first segment **104,** which has a first end and an opposite second end. The first segment **104** is positioned with the first end at the bottom and with the opposite second end at the top. Furthermore, the first segment **104** is arranged at the base **602** with the first end. In one embodiment, the first segment is attached to and fixed to a recess **102** of the base **602,** i.e. the base **602** is provided with a recess **102.** A first end of the first segment **104** of the column **606** is fixed to the base **602** at the recess **102** and recessed into the recess **102.** Thus, the range of reach with the X-ray tube may be extended towards the floor and enable the use of larger front wheels, which improves steering, transportation properties and precision. Furthermore, optimization of the range of reach or the highest possible point with a maintained compact size during transportation may be achieved. Moreover, reduction of the number of components may be achieved, since a sufficient range of reach and a sufficiently high highest possible point can be achieved with only two segments, i.e. the first and second segments, as opposed to three or more segments. Thus, in this embodiment, the front wheels are preferable large.

However, in another embodiment, the first segment is attached to and fixed directly to the base **602,** i.e. the base has no recess **102.**

The second segment **106** is arranged at its first end with an overlap of the first segment's second end and is moveable relative the first segment **104.**

Furthermore, as can be seen from **Fig. 1c****,** the column **606** further has a first elevation adjustment unit, which is adapted to adjust the elevation of the moveable second segment **106.** The first elevation adjustment unit has at least a first threaded elongate member **120.** In a first embodiment, the first threaded elongate member **120** is fixed at the first segment **104.** In this embodiment, a first threaded receiving element **122** is arranged at the second segment **106,** matingly engaged with the threaded elongate member **120.**

In a second embodiment, the first threaded elongate member **120** is fixed at the second segment **106.** In this embodiment, a first threaded receiving element **122** is arranged at the first segment **104,** matingly engaged with the first threaded elongate member **120.**

In other embodiments, the first elevation adjustment unit has a plurality of threaded receiving elements and threaded elongate members.

With the use of a first elevation adjustment unit, high precision elevation adjustments may be obtained.

From **Fig. 1B** position sensors such as limit switches **130, 132** of an elevating column can be seen. Furthermore, the elevating column may have other position sensors. The position sensor may be any device that permits position measurement. Thus, the position sensors may be any of capacitive transducers, capacitive displacement sensors, eddy-current sensors, ultrasonic sensors, grating sensors, Hall effect sensors, inductive non-contact position sensors, Laser Doppler Vibrometers, linear variable differential transformers, multi-axis displacement transducers, photodiode arrays, piezo-electric transducers, proximity sensors, rotary encoders, seismic displacement pick-up sensors or string potentiometers. However, preferably the position sensors are absolute position sensors, such as absolute linear sensors.

In **Fig. 2A** the elevating column **606** is in a position, in which the second segment **106** has been adjusted to a certain height position. From this height position it is possible to move the second segment **106** down or up. Thus, the height position in this figure is an intermediate position.

From **Fig. 2B** some parts of the elevation adjustment unit can be seen. The elevation adjustment unit has in this embodiment a first threaded receiving element **122** and a first threaded elongate member **120.** The first threaded receiving element **122** may be a nut, a nut-shaped element or an elongated hollow rod with an internal thread and the first threaded elongate member **120** may be one or more rods with external threads. The first threaded receiving element **122** is in this embodiment fixed to the first segment **104** with a bracket **210.** The elevation adjustment is in this embodiment made by actuating a rotational movement of the first threaded elongate member **120** with a motor. The motor may be any type of motor, but preferably an electrical motor, such as a Permanent-magnet Direct Current (PMDC) motor or a Brushless Direct Current (BLDC) motor. Other motors that can be used are AC motors or DC motors, such as Induction Motors, Wound Rotor Motors, linear motors or stepper motors.

In another embodiment, the first threaded elongate member **120** may be fixed in a rotational direction and the elevation adjustment performed by actuating a rotational movement of the first threaded receiving element **122** with a motor, preferably an electrical motor, such as a Permanent-magnet Direct Current (PMDC) motor or a Brushless Direct Current (BLDC) motor.

**Fig. 2C** shows a position sensor of the elevating column. The position sensor in this figure is a limit switch **130.** However, also other types of position sensors may be used. Each position sensor gives an indication, preferably an electrical signal, which can be converted to a position and/or a height of the second segment **106** and/or of the column **606.**

**Fig. 3A** shows the elevating column with segments, while the moveable second segment **106** is in its least extended position and the connecting segment **608** is in its highest position. This is the preferred position of the elevating column during transportation of the mobile X-ray apparatus **600,** since the visibility is increased by having the column in an as low position as possible and since the mobile X-ray apparatus **600** is put into an as compact size as possible by positioning the arm **610** on top of the base **602** during transportation.

The connecting element **608** is connected to the second segment **106** and located radially outside the moveable second segment **106.** Since the connecting segment **608** is moveable radially outside the second segment **106** in a vertical direction, the operating elevation range of an arm **610** attached to the connecting element **608** and of image capturing is extended towards very low positions, thus enabling capturing of images at very low positions.

As can be seen from **Fig. 3c,** the connecting segment **608** has an inner part **330** connected to the moveable second segment **106** and an outer part **332** connected to the arm **610,** which may be telescopic. The outer part **332** is rotatable horizontally around the inner part **330.** Thus, the arm **610** may be rotated into an appropriate position and fast and easy rotation of the arm **610** with high precision may be obtained.

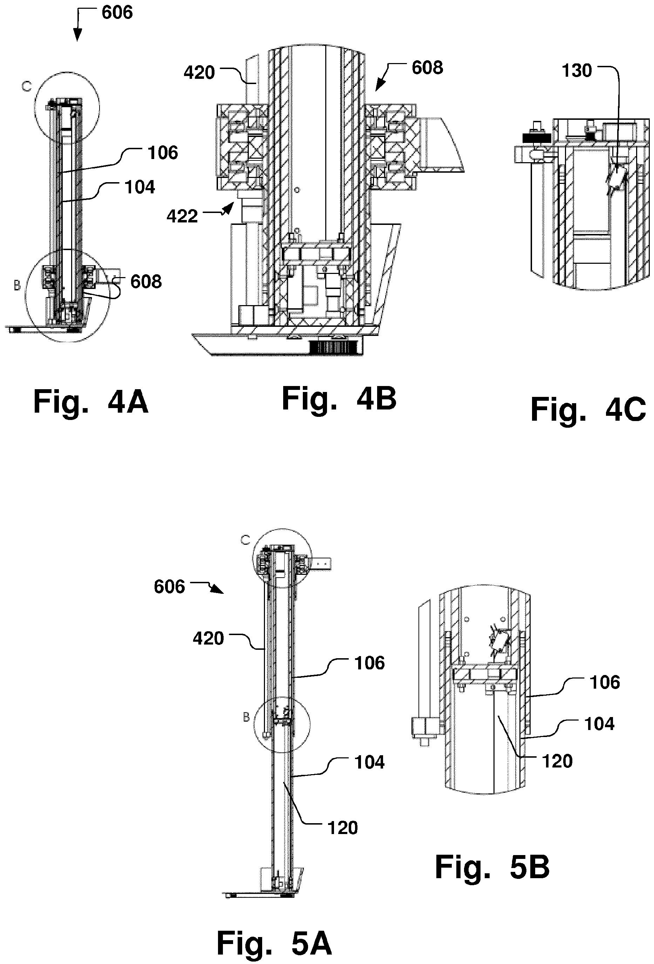

**Fig. 4A** is another view of the elevating column with segments. In this figure, the moveable second segment **106** is in its least extended position, i.e. in the lowest position. Also the connecting segment **608** is in its lowest position. Thus, with the segments in these positions the reach of an X-ray tube attached to the arm **610,** which is attached to the column **606** via a connecting segment **608,** is extended to very low positions close to the floor.

The connecting segment **608,** which is located radially outside the second segment **106,** is moveable in a rotational direction around the second segment **106.** Therefore an arm **610** attached to the connecting segment **608** may be rotated into an appropriate position for capturing of X-ray images. Furthermore, the connecting segment **608** is moveable in a vertical direction along the second segment **106.**

From **Fig. 4B****,** some parts of a second elevation adjustment unit, which is adapted to adjust the elevation of the connecting segment **608,** can be seen. The second elevation adjustment unit has in this embodiment a second threaded receiving element **422** and a second threaded elongate member **420.** The second threaded receiving element **422** may be a nut, a nut-shaped element or an elongated hollow rod with an internal thread and the second threaded elongate member **420** may be a rod with an external thread. The second threaded receiving element **422** is in this embodiment fixed to the connecting segment **608** in a rotational direction or in all directions. The second threaded elongate member **420** is positioned outside the second segment **106** and attached to the second segment **106** at the bottom and the top. E.g. the second threaded elongate member **420** has a first end and an opposite second end. Furthermore, the second threaded elongate member **420** is positioned outside and in parallel with the second segment **106.** Thus the first end of the second threaded elongate member **420** is positioned at the first end of the second segment **106** and the second end of the second threaded elongate member **420** is positioned at the second end of the second segment **106.**

Alternatively, the threaded elongate member **420** may be positioned inside the second segment **106,** e.g. if there is a slot in the second segment **106** and/or the second segment **106** may be used as a cover for the threaded elongate member **420.**

The elevation adjustment is in this embodiment made by actuating a rotational movement of the second threaded elongate member **420** with a motor. The motor may be any type of motor, but preferably an electrical motor, such as a Permanent-magnet Direct Current (PMDC) motor or a Brushless Direct Current (BLDC) motor. Other motors that can be used are AC motors or DC motors, such as Induction Motors, Wound Rotor Motors, linear motors or stepper motors. In this embodiment, the second threaded receiving element **422** is arranged at the connecting segment **608** and fixed thereto. It is matingly engaged with the threaded elongate member **420.**

In another embodiment, the second threaded elongate member **420** may be fixed in a rotational direction and the elevation adjustment performed by actuating a rotational movement of the second threaded receiving element **422** with a motor, preferably an electrical motor, such as a Permanent-magnet Direct Current (PMDC) motor or a Brushless Direct Current (BLDC) motor. The motor is preferably positioned inside the moveable second segment **106.** In this embodiment, the second threaded elongate member **120** is fixed at the connecting segment **608.** Furthermore, in this embodiment, a second threaded receiving element **122** is arranged at the second segment **106,** matingly engaged with the second threaded elongate member **420.**

By the use of a first and a second elevation adjustment unit, elevation adjustment is split between adjusting elevation of the second segment **106** and adjusting elevation of the connecting segment **608.** Thus, faster elevation adjustments and/or elevation adjustments with higher precision may be achieved.

A control unit may be used for calculating a first, a second and/or a total amount of elevation adjustment. The calculations of these amounts may be based on a measured present elevation of the connecting element **608** and/or the second segment **106.** Furthermore the calculations may also be based on a reference value or a desired value, which may be preset or supplied by the user. The control unit may also be used to control a second actuator, such as a second electrical motor, to make the second elevation adjustment unit move the connecting segment **608** to an elevation depending on the second amount of elevation adjustment. Furthermore, the control unit may be used to control a first actuator, such as a first electrical motor, to make the first elevation adjustment unit move the second segment **106** to an elevation depending on the first amount of elevation adjustment. Thereby precision and/or speed of elevation adjustment may be increased. With this arrangement, it is also possible to operate and move the connecting segment **608** in an opposite direction as movement of the moveable second segment **106.** This will be the case when one of the first and second amounts of elevation adjustment is negative and the other of the first and second amounts of elevation adjustment is positive.

Furthermore, the first actuator or electrical motor may be fixed to the base. It may be located at a distance from the elevating column **606.** Moreover, it may be connected to the first elevation adjustment unit with a chain, cable or a belt. Thus, space inside the column **606** is freed up and can be used for other purposes. Furthermore, the column may be made smaller, i.e. with a smaller circumference or cross section. Moreover, the centre of gravity may be made lower with the motor outside the column **606** instead of inside the column **606.** Thus the weight may be lowered.

From **Fig. 4C** a limit switch **130** of the elevating column **606** can be seen. Furthermore, the elevating column **606** may also have other position sensors. The moveable second segment **106** is in this figure in its least extended position and the connecting segment **608** in its lowest position. Since the connecting segment **608** is in its lowest position, it should not be able to move any lower. This can be ensured by having some kind of stop plates or limit switches **130, 132,** which ensures that the connecting segment **608** cannot move past a lowest and/or highest position. Alternatively, or in addition, the connecting segment **608** may be controlled to move only within a certain range of movement and automatically stop when the position measured with position sensors (not shown) reaches a lowest or highest allowed value. Such a control can be made with a control unit, which sends signals to the movement actuators. The movement of the second segment **106** may be controlled the same way or in a similar way. The control of movement of the second segment **106** may then utilize information retrieved from the position sensors. Furthermore, the second and first threaded elongate members may have automatic braking properties.

As can be seen from **Figs. 5A** and **5B****,** the first threaded elongate member **120** has a first end and an opposite second end. The first threaded elongate member **120** is positioned inside the first segment **104** so that the first end of the first threaded elongate member **120** is positioned at the first end of the first segment **104** and the second end of the first threaded elongate member **120** is positioned at the second end of the first segment **104.** In this embodiment, a u-shaped profile is attached to and positioned inside the second segment **106.** Furthermore, the first threaded receiving element **122** is fixed to the u-shaped profile. The use of the u-shaped profile increases stability and avoids wobbling of the first threaded elongate member **120** and thus increases precision, reliability, safety and life span of the threaded elongate member **120,** the first elevation adjustment unit and the system.

Alternatively, the first threaded elongate member **120** is positioned inside the second segment **106** so that the first end of the first threaded elongate member **120** is positioned at the first end of the second segment **106** and the second end of the first threaded elongate member **120** is positioned at the second end of the second segment **106.** In this embodiment, a u-shaped profile is attached to and positioned inside the first segment **104.** Furthermore, the first threaded receiving element **122** is fixed to the u-shaped profile.

In one embodiment, a pitch or lead of an internal thread of the second threaded receiving element **422** and an external thread of the second threaded elongate member **420** is larger than a pitch or lead of an internal thread of the first threaded receiving element **122** and an external thread of the first threaded elongate member **120,** with e.g. a factor of 2. Thus, the moveable second segment can be height adjusted fast and the connecting segment can be used for alignment with high precision.

Furthermore, the second segment **106** and/or the first segment **104** may comprise four complete walls arranged in a rectangular or square formation cross-sectionally. The walls may be made without any slots and consequently the walls may be made thinner than if slots had been present. Thus the weight may be lowered and/or the stability of the column **606** improved. Alternatively, at least one of the walls may have at least one slot.

Moreover, the elevating column **606** may be operated without a counter-weight. This enables a compact size during transportation, and thus enables the use of a smaller vehicle, such as a van for transportation. Therefore cheaper transportation and lower fuel consumption may be achieved.

In one embodiment, the overlap of the second and first segments **106, 104,** when the column **606** is set to a highest position, is optimal and within 10-20 cm, and preferably within 15-17 cm.

In another embodiment, a ratio between an overlap of the second and first segments **106, 104,** when the column **606** is set to a highest position, and a maximum height above the floor of the column **606** is below 0.1 and preferably below 0.075.

In yet another embodiment, a length of the second segment **106** and a length of the first segment **104** are selected so that a span between a lowest possible position and a highest possible position of the elevating column **606** is optimized and at least 1.5 m, and preferably at least 1.7 m.

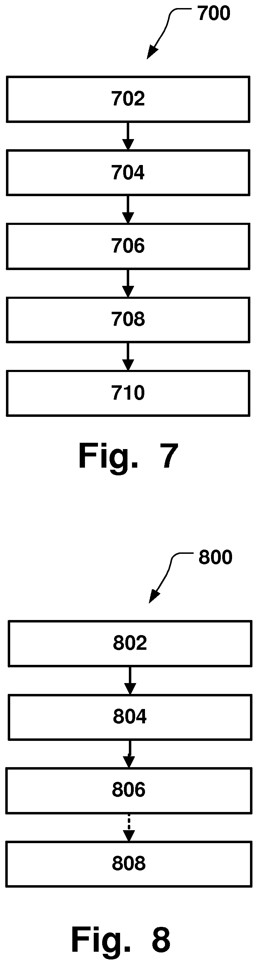

**Fig. 7** shows some steps of a method **700** of controlling elevation of the elevating column **606.** From this figure it can be seen that an elevation of a moveable second segment **106** of the elevating column **606** is controlled **706** with a first actuator, such as a first electrical motor, e.g. a Permanent-magnet Direct Current (PMDC) motor or a Brushless Direct Current (BLDC) motor. A first control signal is sent **704** from a control unit, which has received **702** information from position sensors, to the first actuator. The first actuator actuates a rotational movement of either a first threaded receiving element **122** or a first threaded elongate member **120** of a first elevation adjustment unit. Thus, the actuator actuates an elevation adjustment of the second segment **106.**

Furthermore, an elevation of a connecting segment **608,** which is preferably attached to a telescopic arm **610,** of the elevating column **606** is controlled **710** with a second actuator, such as a second electrical motor, e.g. a Permanent-magnet Direct Current (PMDC) motor or a Brushless Direct Current (BLDC) motor. A second control signal is sent **708** from a control unit, which has received **702** information from position sensors, to the second actuator. The second actuator actuates a rotational movement of either a second threaded receiving element **422** or a second threaded elongate member **420** of a second elevation adjustment unit. Thus, the second actuator actuates an elevation adjustment of the connecting segment **608.**

With this method, a fast and easy control of the elevation of the elevating column **606** may be achieved. The elevation of the moveable second segment **106** and the elevation of the connecting segment **608** are in one embodiment controlled simultaneously. Thus, the speed of adjustment may be increased.

**Fig. 8** shows some steps of a method **800** of preparing a mobile X-ray apparatus **600** for transport. In **802,** control of elevation of a moveable second segment of an elevating column to a lowest possible position with a first actuator, such as a first electrical motor, e.g. a Permanent-magnet Direct Current (PMDC) motor or a Brushless Direct Current (BLDC) motor, is performed. A first control signal is sent from a control unit, which may have received information from position sensors, to the first actuator. The first actuator actuates a rotational movement of either a first threaded receiving element **122** or a first threaded elongate member **120** of a first elevation adjustment unit. Thus, the actuator actuates an elevation adjustment of the second segment **106** so that the second segment **106** reaches the lowest possible position.

Furthermore, in **804** control of elevation of a connecting segment **608,** comprising an outer part **332** and an inner part **330** attached to an arm **610,** which may be telescopic, of the elevating column **606** to a highest possible position with a second actuator, such as a second electrical motor, e.g. a Permanent-magnet Direct Current (PMDC) motor or a Brushless Direct Current (BLDC) motor, is performed. A second control signal is sent from a control unit, which has received information from position sensors, to the second actuator. The second actuator actuates a rotational movement of either a second threaded receiving element **422** or a second threaded elongate member **420** of a second elevation adjustment unit. Thus, the second actuator actuates an elevation adjustment of the connecting segment **608** so that the connecting segment **608** reaches a highest possible position.

Moreover, in **806,** rotation of the outer part **332** horizontally around the inner part **330** until an arm **610,** which may be telescopic, of the apparatus is positioned on top of a base **602** of the apparatus, is performed. Optionally, in **808,** control of elevation of the connecting segment **608** by lowering it until the arm **610** is positioned in a locking position, such as in a hollow space of the base **602,** is performed. With the use of this method, a compact size during transportation is enabled. Furthermore, the mobile X-ray apparatus may be easy to transport due to e.g. compact size and increased visibility.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. The present disclosure has been described above with reference to specific embodiments. However, other embodiments than the above described are equally possible within the scope of the disclosure. Different method steps or a different order thereof than those described above may be provided within the scope of the disclosure. The different features and steps of the disclosure may be combined in other combinations than those described. The scope of the disclosure is only limited by the appended patent claims.

## Claims

1. A mobile X-ray apparatus (600) comprising an elevating column, said column (606) preferably being rotationally fixed in relation to a base (602) of said X ray apparatus (600), and said column (606) having a plurality of segments, including:
a first segment (104), having a first end and an opposite second end, arranged at said base (602) with said first end, and
a second segment (106) having a first end and an opposite second end;
wherein said second segment (106) is arranged at its first end with an overlap of said first segment's second end and is moveable relative said first segment (104); and
said column (606) further including a first elevation adjustment unit including:
a first threaded elongate member (120) fixed at one of said first and second segments (104, 106), and
a first threaded receiving element (122) arranged at the other of said first and second segments (104, 106), matingly engaged with said threaded elongate member (120) for elevation adjustment of said column (606),
the elevating column further including a connecting segment (608) attaching an arm (610) holding an X-ray tube to said elevating column,
the mobile X-ray apparatus being **characterized by** said connecting segment (608) being located radially outside said second segment (106) and connected thereto, wherein said connecting segment (608) is movable radially outside said second segment (106) in a vertical direction.

2. The mobile X-ray apparatus (600) according to claim 1, wherein said moveable second segment is positioned in its lowest position and said connecting segment is positioned at the top of said moveable second segment during transportation of said apparatus.

3. The mobile X-ray apparatus (600) according to any one of claims 1-2, wherein said arm is a telescopic arm.

4. The mobile X-ray apparatus (600) according to claim 3, wherein said connecting segment includes:
an outer part connected to said telescopic arm, and
an inner part connected to said moveable second segment; and
wherein said outer part is rotatable horizontally around said inner part.

5. The mobile X-ray apparatus (600) according to any one of claims 1-4, further including a second elevation adjustment unit including:
a second threaded receiving element arranged at one of said second segment and said connecting segment, and
at least a second threaded elongate member fixed at the other of said second segment and said connecting segment, matingly engaged with said second threaded receiving element; and
wherein said first elevation adjustment unit is adapted to adjust the elevation of said moveable second segment and said second elevation adjustment unit is adapted to adjust the elevation of said connecting segment.

6. The mobile X-ray apparatus (600) according to claim 5, wherein an elevation adjustment performed by said first elevation adjustment unit is actuated by a first electrical motor, such as a Permanent-magnet Direct Current (PMDC) motor or a Brushless Direct Current (BLDC) motor, connected to one of said first threaded receiving element and said first threaded elongate member and an elevation adjustment performed by said second elevation adjustment unit is actuated by a second electrical motor, such as a Permanent-magnet Direct Current (PMDC) motor or a Brushless Direct Current (BLDC) motor, preferably positioned inside said moveable second segment and connected to one of said second threaded receiving element and said second threaded elongate member.

7. A method of controlling elevation of the elevating column of the mobile X-ray apparatus of any of claims 1-4 including:
receiving information from position sensors,
sending a first control signal to a first electrical motor,
controlling an elevation of the moveable second segment of said elevating column with said first electrical motor by actuating a rotational movement of either the first threaded receiving element or the first threaded elongate member of the first elevation adjustment unit,
sending a second control signal to a second electrical motor,
controlling an elevation of the connecting segment, attached to the arm holding the X-ray tube, of said elevating column with a second electrical motor by actuating a rotational movement of either a second threaded receiving element or a second threaded elongate member of a second elevation adjustment unit.

8. The method of claim 7, wherein said elevation of said moveable second segment and said elevation of said connecting segment are controlled simultaneously.

9. A method of preparing the mobile X-ray apparatus of any of claims 1-3, for transport, including:
controlling elevation of the moveable second segment of the elevating column to a lowest possible position with a first electrical motor by actuating a rotational movement of either the first threaded receiving element or the first threaded elongate member of a first elevation adjustment unit,
controlling elevation of the connecting segment, comprising an outer part and an inner part attached to the arm holding the X-ray tube, of said elevating column to a highest possible position with a second electrical motor by actuating a rotational movement of either a second threaded receiving element or a second threaded elongate member of a second elevation adjustment unit,
rotating said outer part horizontally around said inner part until said arm is positioned on top of a base of said apparatus, and
optionally controlling elevation of said connecting segment by lowering it until said arm is positioned in a locking position, such as in a hollow space of said base.

10. The mobile X-ray apparatus (600) according to any one of claims 5-6 or the method of any one of claims 7-9, wherein said second threaded elongate member has a first end and an opposite second end and wherein said second threaded elongate member is positioned outside and in parallel with said second segment so that said first end of said second threaded elongate member is positioned at said first end of said second segment and said second end of said second threaded elongate member is positioned at said second end of said second segment.

11. The mobile X-ray apparatus (600) or the method according to claim 10, wherein said first threaded elongate member has a first end and an opposite second end and wherein said first threaded elongate member is positioned inside one of said first and second segments so that said first end of said first threaded elongate member is positioned at said first end of said first or second segment and said second end of said first threaded elongate member is positioned at said second end of said first or second segment.

12. The mobile X-ray apparatus (600) or the method according to claim 11, wherein a u-shaped profile is attached to and positioned inside one of said first and second segments and wherein said first threaded receiving element is fixed to said u-shaped profile.

13. The mobile X-ray apparatus (600) according to any one of claims 6 or 10-12 or the method according to any of claims 7-12, wherein at least one of said second and first threaded elongate members has automatic braking properties.

14. The mobile X-ray apparatus (600) according to any of claims 1-6 or 10-13 or the method according to any one of claims 7-13, wherein said base is provided with a recess and wherein said first end of said first segment is fixed to said base at said recess and recessed into said recess.

## Patentansprüche

1. Mobile Röntgenvorrichtung (600), umfassend eine Hubsäule, wobei die Hubsäule (606) bevorzugt in Bezug auf eine Basis (602) der Röntgenvorrichtung (600) drehfest befestigt ist und die Hubsäule (606) mehrere Segmente aufweist, umfassend:
ein erstes Segment (104), das ein erstes Ende und ein entgegengesetztes zweites Ende aufweist und mit dem ersten Ende an der Basis (602) angeordnet ist, und
ein zweites Segment (106), das ein erstes Ende und ein entgegengesetztes zweites Ende aufweist;
wobei das zweite Segment (106) an seinem ersten Ende mit einer Überlappung des zweiten Endes des ersten Segments angeordnet und relativ zu dem ersten Segment (104) beweglich ist; und
die Hubsäule (606) ferner eine erste Höhenanpassungseinheit umfasst, die das Folgende umfasst:
ein erstes längliches Gewindeelement (120), das an einem von dem ersten und dem zweiten Segment (104, 106) befestigt ist, und
ein erstes Gewindeaufnahmeelement (122), das an dem anderen von dem ersten und dem zweiten Segment (104, 106) angeordnet ist und mit dem länglichen Gewindeelement (120) zur Höhenanpassung der Hubsäule (606) passend in Eingriff steht,
wobei die Hubsäule ferner ein Verbindungssegment (608) umfasst, das einen Arm (610) an der Hubsäule befestigt, der eine Röntgenröhre hält, und die mobile Röntgenvorrichtung **dadurch gekennzeichnet ist, dass** das Verbindungssegment (608) radial außerhalb des zweiten Segments (106) angeordnet und damit verbunden ist, wobei das Verbindungssegment (608) radial außerhalb des zweiten Segments (106) in einer vertikalen Richtung beweglich ist.

2. Mobile Röntgenvorrichtung (600) nach Anspruch 1, wobei das bewegliche zweite Segment in seiner niedrigsten Position positioniert ist und das Verbindungssegment an der Oberseite des beweglichen zweiten Segments positioniert ist während des Transports der Vorrichtung.

3. Mobile Röntgenvorrichtung (600) nach einem der Ansprüche 1 bis 2, wobei der Arm ein Teleskoparm ist.

4. Mobile Röntgenvorrichtung (600) nach Anspruch 3, wobei das Verbindungssegment das Folgende umfasst:
einen äußeren Teil, der mit dem Teleskoparm verbunden ist, und
einen inneren Teil, der mit dem beweglichen zweiten Segment verbunden ist; und
wobei der äußere Teil horizontal um den inneren Teil drehbar ist.

5. Mobile Röntgenvorrichtung (600) nach einem der Ansprüche 1 bis 4, ferner umfassend eine zweite Höhenanpassungseinheit, die das Folgende umfasst:
ein zweites Gewindeaufnahmeelement, das an einem von dem zweiten Segment und dem Verbindungssegment angeordnet ist, und
mindestens ein zweites längliches Gewindeelement, das an dem anderen von dem zweiten Segment und dem Verbindungssegment befestigt ist und mit dem zweiten Gewindeaufnahmeelement passend in Eingriff steht; und
wobei die erste Höhenanpassungseinheit angepasst ist, die Höhe des beweglichen zweiten Segments anzupassen, und die zweite Höhenanpassungseinheit angepasst ist, die Höhe des Verbindungssegments anzupassen.

6. Mobile Röntgenvorrichtung (600) nach Anspruch 5, wobei eine von der ersten Höhenanpassungseinheit ausgeführte Höhenanpassung durch einen ersten Elektromotor, wie beispielsweise einen Permanentmagnetgleichstrommotor (PMDC-Motor) oder einen bürstenlosen Gleichstrommotor (BLDC-Motor), betätigt wird, der mit einem von dem ersten Gewindeaufnahmeelement und dem ersten länglichen Gewindeelement verbunden ist, und eine von der zweiten Höhenanpassungseinheit ausgeführte Höhenanpassung von einem zweiten Elektromotor, wie beispielsweise einem Permanentmagnetgleichstrommotor (PMDC-Motor) oder einem bürstenlosen Gleichstrommotor (BLDC-Motor), betätigt wird, der bevorzugt innerhalb des beweglichen zweiten Segments positioniert und mit einem von dem zweiten Gewindeaufnahmeelement und dem zweiten länglichen Gewindeelement verbunden ist.

7. Verfahren zum Steuern der Höhe der Hubsäule der mobilen Röntgenvorrichtung nach einem der Ansprüche 1 bis 4, umfassend:
Empfangen von Informationen von Positionssensoren,
Senden eines ersten Steuersignals an einen ersten Elektromotor,
Steuern einer Höhe des beweglichen zweiten Segments der Hubsäule mit dem ersten Elektromotor durch Betätigung einer Drehbewegung von entweder dem Gewindeaufnahmeelement oder dem ersten länglichen Gewindeelement der ersten Höhenanpassungseinheit,
Senden eines zweiten Steuersignals an einen zweiten Elektromotor,
Steuern einer Höhe des an dem Arm, der die Röntgenröhre hält, befestigten Verbindungssegments der Hubsäule mit einem zweiten Elektromotor durch Betätigung einer Drehbewegung von entweder einem zweiten Gewindeaufnahmeelement oder einem zweiten länglichen Gewindeelement einer zweiten Höhenanpassungseinheit.

8. Verfahren nach Anspruch 7, wobei die Höhe des beweglichen zweiten Segments und die Höhe des Verbindungssegments gleichzeitig gesteuert werden.

9. Verfahren zum Vorbereiten der mobilen Röntgenvorrichtung nach einem der Ansprüche 1 bis 3 für den Transport, umfassend:
Steuern der Höhe des beweglichen zweiten Segments der Hubsäule in eine niedrigstmögliche Position mit einem ersten Elektromotor durch Betätigung einer Drehbewegung von entweder dem Gewindeaufnahmeelement oder dem ersten länglichen Gewindeelement einer ersten Höhenanpassungseinheit,
Steuern der Höhe des Verbindungssegments der Hubsäule, das einen äußeren Teil und einen inneren Teil umfasst, befestigt an dem Arm, der die Röntgenröhre hält, in eine höchstmögliche Position mit einem zweiten Elektromotor durch Betätigung einer Drehbewegung von entweder einem zweiten Gewindeaufnahmeelement oder einem zweiten länglichen Gewindeelement einer zweiten Höhenanpassungseinheit,
Drehen des äußeren Teils horizontal um den inneren Teil, bis der Arm oben auf einer Basis der Vorrichtung positioniert ist, und
optional Steuern der Höhe des Verbindungssegments durch Absenken dessen, bis der Arm in einer Arretierungsposition, wie beispielsweise in einem Hohlraum der Basis, positioniert ist.

10. Mobile Röntgenvorrichtung (600) nach einem der Ansprüche 5 bis 6 oder Verfahren nach einem der Ansprüche 7 bis 9, wobei das zweite längliche Gewindeelement ein erstes Ende und ein entgegengesetztes zweites Ende aufweist, und wobei das zweite längliche Gewindeelement außerhalb und parallel zu dem zweiten Segment positioniert ist, sodass das erste Ende des zweiten länglichen Gewindeelements an dem ersten Ende des zweiten Segments positioniert ist und das zweite Ende des zweiten länglichen Gewindeelements an dem zweiten Ende des zweiten Segments positioniert ist.

11. Mobile Röntgenvorrichtung (600) oder Verfahren nach Anspruch 10, wobei das erste längliche Gewindeelement ein erstes Ende und ein entgegengesetztes zweites Ende aufweist, und wobei das erste längliche Gewindeelement innerhalb eines von dem ersten und dem zweiten Segment positioniert ist, sodass das erste Ende des ersten länglichen Gewindeelements am ersten Ende des ersten oder zweiten Segments positioniert ist und das zweite Ende des ersten länglichen Gewindeelements an dem zweiten Ende von dem ersten oder zweiten Segment positioniert ist.

12. Mobile Röntgenvorrichtung (600) oder Verfahren nach Anspruch 11, wobei ein u-förmiges Profil an einem von dem ersten und dem zweiten Segment angebracht und innerhalb davon positioniert ist, und wobei das erste Gewindeaufnahmeelement an dem u-förmigen Profil befestigt ist.

13. Mobile Röntgenvorrichtung (600) nach einem der Ansprüche 6 oder 10 bis 12 oder Verfahren nach einem der Ansprüche 7 bis 12, wobei mindestens eines von dem zweiten und dem ersten länglichen Gewindeelement automatische Bremseigenschaften aufweist.

14. Mobile Röntgenvorrichtung (600) nach einem der Ansprüche 1 bis 6 oder 10 bis 13 oder Verfahren nach einem der Ansprüche 7 bis 13, wobei die Basis mit einer Aussparung versehen ist, und wobei das erste Ende des ersten Segments an der Basis an der Aussparung befestigt und in die Aussparung versenkt ist.

## Revendications

1. Appareil de radiographie mobile (600) comprenant une colonne élévatrice, ladite colonne (606) étant de préférence fixée en rotation par rapport à une base (602) dudit Appareil de radiographie(600), et ladite colonne (606) ayant une pluralité de segments, incluant :
un premier segment (104), ayant une première extrémité et une deuxième extrémité opposée, disposé au niveau de ladite base (602) avec ladite première extrémité, et
un deuxième segment (106) ayant une première extrémité et une deuxième extrémité opposée ;
dans lequel ledit deuxième segment (106) est agencé à sa première extrémité avec un chevauchement de la deuxième extrémité dudit premier segment et est mobile par rapport audit premier segment (104) ; et
ladite colonne (606) comprenant en outre une première unité de réglage d'élévation comprenant :
un premier élément allongé fileté (120) fixé à l'un desdits premier et deuxième segments (104, 106), et
un premier élément de réception fileté (122) agencé à l'autre desdits premier et deuxième segments (104, 106), mis en prise par emboîtement avec ledit élément allongé fileté (120) pour le réglage en élévation de ladite colonne (606),
la colonne élévatrice comprenant en outre un segment de raccordement (608) fixant un bras (610) maintenant un tube à rayons X à ladite colonne élévatrice, l'appareil de radiographie mobile étant **caractérisé en ce que** ledit segment de raccordement (608) est situé radialement à l'extérieur dudit deuxième segment (106) et raccordé à celui-ci, dans lequel ledit segment de raccordement (608) est mobile radialement à l'extérieur dudit deuxième segment (106) dans une direction verticale.

2. Appareil de radiographie mobile (600) selon la revendication 1, dans lequel ledit deuxième segment mobile est positionné dans sa position la plus basse et ledit segment de raccordement est positionné en haut dudit deuxième segment mobile lors du transport dudit appareil.

3. Appareil de radiographie mobile (600) selon une quelconque des revendications 1 à 2, dans lequel ledit bras est un bras télescopique.

4. Appareil de radiographie mobile (600) selon la revendication 3, dans lequel ledit segment de raccordement comprend :
une partie externe raccordée audit bras télescopique, et
une partie interne raccordée audit deuxième segment mobile ; et
dans lequel ladite partie extérieure peut tourner horizontalement autour de ladite partie intérieure.

5. Appareil de radiographie mobile (600) selon une quelconque des revendications 1 à 4, comprenant en outre une deuxième unité de réglage d'élévation comprenant :
un deuxième élément de réception fileté agencé au niveau d'un dudit deuxième segment et dudit segment de raccordement, et
au moins un deuxième élément allongé fileté fixé à l'autre desdits un deuxième segment et dudit segment de raccordement, mis en prise par emboîtement avec ledit deuxième élément de réception fileté ; et
dans lequel ladite première unité de réglage d'élévation est adaptée pour ajuster l'élévation dudit deuxième segment mobile et ladite deuxième unité de réglage d'élévation est adaptée pour ajuster l'élévation dudit segment de raccordement.

6. Appareil de radiographie mobile (600) selon la revendication 5, dans lequel un réglage d'élévation effectué par ladite première unité de réglage d'élévation est actionné par un premier moteur électrique, tel qu'un moteur à courant continu à aimant permanent (PMDC) ou un moteur sans balais à courant continu (BLDC), raccordé à un dudit premier élément de réception fileté et dudit premier élément allongé fileté et un réglage d'élévation effectué par ladite deuxième unité de réglage d'élévation est actionné par un deuxième moteur électrique, tel qu'un courant continu à aimant permanent (PMDC) ou un moteur sans balais à courant continu (BLDC), positionné de préférence à l'intérieur dudit deuxième segment mobile et raccordé à un dudit deuxième élément récepteur fileté et dudit deuxième élément allongé fileté.

7. Procédé de commande de l'élévation de la colonne élévatrice de l'appareil de radiographie mobile selon une quelconque des revendications 1 à 4, comprenant :
la réception des informations de capteurs de position,
l'envoi d'un premier signal de commande à un premier moteur électrique,
la commande d'une élévation du deuxième segment mobile de ladite colonne élévatrice avec ledit premier moteur électrique en actionnant un mouvement de rotation soit du premier élément de réception fileté, soit du premier élément fileté allongé de la première unité de réglage d'élévation,
l'envoi d'un deuxième signal de commande à un deuxième moteur électrique,
la commande d'une élévation du segment de raccordement, fixé au bras maintenant le tube à rayons X, de ladite colonne élévatrice avec un deuxième moteur électrique en actionnant un mouvement de rotation soit d'un deuxième élément de réception fileté, soit d'un deuxième élément allongé fileté d'une deuxième unité de réglage d'élévation.

8. Procédé selon la revendication 7, dans lequel ladite élévation dudit deuxième segment mobile et ladite élévation dudit segment de raccordement sont commandées simultanément.

9. Procédé de préparation de l'appareil de radiographie mobile selon une quelconque des revendications 1 à 3, pour le transport, comprenant :
la commande de l'élévation du deuxième segment mobile de la colonne élévatrice à une position la plus basse possible avec un premier moteur électrique en actionnant un mouvement de rotation soit du premier élément de réception fileté soit du premier élément allongé fileté d'une première unité de réglage d'élévation ;
la commande de l'élévation du segment de raccordement, comprenant une partie extérieure et une partie intérieure fixées au bras maintenant le tube à rayons X, de ladite colonne élévatrice à une position la plus élevée possible avec un deuxième moteur électrique en actionnant un mouvement de rotation soit d'un deuxième élément récepteur fileté, soit d'un deuxième élément allongé fileté d'une deuxième unité de réglage d'élévation,
la rotation de ladite partie extérieure horizontalement autour de ladite partie intérieure jusqu'à ce que ledit bras soit positionné au-dessus d'une base dudit appareil, et
la commande éventuellement l'élévation dudit segment de raccordement en l'abaissant jusqu'à ce que ledit bras soit positionné dans une position de verrouillage, telle que dans un espace creux dudit socle.

10. Appareil de radiographie mobile (600) selon une quelconque des revendications 5 à 6 ou procédé selon une quelconque des revendications 7 à 9, dans lequel ledit deuxième élément allongé fileté possède une première extrémité et une deuxième extrémité opposée et dans lequel ledit deuxième élément allongé fileté est positionné à l'extérieur et parallèlement audit deuxième segment de sorte que ladite première extrémité dudit deuxième élément allongé fileté soit positionnée à ladite première extrémité dudit deuxième segment et ladite deuxième extrémité dudit deuxième élément allongé fileté soit positionnée à ladite deuxième extrémité dudit deuxième segment.

11. Appareil de radiographie mobile (600) ou procédé selon la revendication 10, dans lequel ledit premier élément allongé fileté possède une première extrémité et une deuxième extrémité opposée et dans lequel ledit premier élément allongé fileté est positionné à l'intérieur d'un desdits premier et deuxième segments de sorte que ladite première extrémité dudit premier élément allongé fileté soit positionnée à ladite première extrémité dudit premier ou deuxième segment et ladite deuxième extrémité dudit premier élément allongé fileté soit positionnée à ladite deuxième extrémité dudit premier ou deuxième segment.

12. Appareil de radiographie mobile (600) ou procédé selon la revendication 11, dans lequel un profilé en forme de U est fixé à et positionné à l'intérieur d'un desdits premier et deuxième segments et dans lequel ledit premier élément de réception fileté est fixé audit profilé en U.

13. Appareil de radiographie mobile (600) selon une quelconque des revendications 6 ou 10 à 12 ou procédé selon une quelconque des revendications 7 à 12, dans lequel au moins un desdits deuxième et premier éléments allongés filetés a des propriétés de freinage automatique.

14. Appareil de radiographie mobile (600) selon une quelconque des revendications 1 à 6 ou 10 à 13 ou procédé selon une quelconque des revendications 7 à 13, dans lequel ladite base est pourvue d'un évidement et dans lequel ladite première extrémité dudit premier segment est fixée à ladite base au niveau dudit évidement et enfoncée dans ledit évidement.
